# EUROPEAN PATENT SPECIFICATION

(11) **EP 1 797 431 B1**
(45) Date of publication and mention of the grant of the patent: **26.01.2011**
(21) Application number: 05807568.0
(22) Date of filing: 06.10.2005
(51) Int. Cl.: G01N 33/53

(54) **DETECTION OF ELEVATED LEVELS OF HER-2/NEU PROTEIN ON CIRCULATING CANCER CELLS AND TREATMENT**
ERKENNUNG EINES ERHÖHTEN SPIEGELS VON HER-2/NEU-PROTEINEN IN ZIRKULIERENDEN KREBSZELLEN UND DESSEN BEHANDLUNG
DETECTION DE TAUX ELEVES DE PROTEINE HER-2/NEU SUR DES CELLULES CANCEREUSES CIRCULANTES ET TRAITEMENT ASSOCIE

(30) Priority: 06.10.2004 US 616332 P
(43) Date of publication of application: 20.06.2007
(73) Proprietor: Wellstat Biologics Corporation, Gaithersburg, MD 20878 (US)
(72) Inventor: LORENCE, Robert, M., Bethesda, MD 20817 (US); LU, Ming, Potomac, MD 20854 (US)
(74) Representative: MacLean, Martin Robert
(86) International application number: PCT/US2005/035894
(87) International publication number: WO 2006/041959

(56) References cited:
- WO-A-00/52474
- WO-A-03/065042
- WO-A-2004/008099
- US-A- 5 401 638
- US-A- 5 604 107
- BLACKBURN G F ET AL: "ELECTROCHEMILUMINESCENCE DETECTION FOR DEVELOPMENT OF IMMUNOASSAYS AND DNA PROBE ASSAYS FOR CLINICAL DIAGNOSTICS" CLINICAL CHEMISTRY, AMERICAN ASSOCIATION FOR CLINICAL CHEMISTRY, WASHINGTON, DC, vol. 37, no. 9, 1 September 1991 (1991-09-01), pages 1534-1539, XP000232533 ISSN: 0009-9147
- STEARNS ET AL.: 'Circulating tumor markers in breast cancer: accepted utilities and novel prospects' BREAST CANCER RESEARCH AND TREATMENT vol. 52, 1998, pages 239 - 259, XP008095781
- BASELGA: 'Is circulating NER-2 more than just a tumor marker?' CLINICAL CANCER RESEARCH vol. 7, 2001, pages 2605 - 2607, XP008095820
- KOSTLER ET AL.: 'Monitoring of serum Her-2/neu predicts response and progression-free survival to trastuzumab-based treatment in patients with metastatic breast cancer' CLINICAL CANCER RESEARCH vol. 10, pages 1618 - 1624, XP002378020
- MAPLE ET AL.: 'Development and validation of ELISA for Herceptin detection in human serum' J. OF IMMUNOLOGICAL METHODS vol. 295, 2004, pages 169 - 182, XP004701203
- LIPTON ET AL.: 'Elevated serum HER-2/neu level predicts decreased response to hormone therapy in metastatic breast cancer' J. OF CLINICAL ONCOLOGY vol. 20, 2002, pages 1467 - 1472, XP008096329
- FORUS ET AL.: 'Sensitive fluorescent in situ hybridisation method for the characterisation of breast cancer cells in bone marrow aspirates' J. CLIN. PATHOL.: MOL. PATHOL. vol. 52, 1999, pages 68 - 74, XP008096330
- HAGER ET AL.: 'The use of a panel of monoclonal antibodies to enrich circulating breast cancer cells facilitates their detection' GYNECOLOGIC ONCOLOGY vol. 98, 2005, pages 211 - 216, XP004985051
- COOK ET AL.: 'Clinical Utility of Serum HER-2/neu testing on the Bayer Immunol (R) automated system in breast cancer' ANTICANCER RESEARCH vol. 21, 2001, pages 1465 - 4470, XP008095778

## Description

### BACKGROUND OF THE INVENTION

Currently only 25 to 30% of breast cancer patients receive HERCEPTIN therapy based on the findings of elevated levels of the Her-2/neu (also called Her2/neu; HER2; c-erbB-2 and erbB2) protein or gene in biopsies of their primary tumor. The data in the literature suggest that a significant number of women (11 of 26 tested) with negative results for Her2/neu in their primary tumor biopsy go on to develop Her2/neu positivity on their circulating cancer cells (Hayes DF, et al., Int J Oncol 21:1111-7; Meng S et al., 2004 Proc Natl Acad Sci USA 101:9393-98). Also, a considerable number of women with breast cancer do not have biopsy material readily available for testing for Her-2-neu status. The approaches used in the papers by Hayes et al and by Meng et al are cumbersome and time-consuming and a rapid more convenient test is needed, especially one that can be done in the physician's office. The method of Hayes et al. requires flow cytometric analysis and the method of Meng et al. requires fluorescence in situ hybridization (FISH) which are more complicated and time-consuming than direct detection (e.g., by ECL) of Her-2/neu protein as noted in this invention.

Her2/neu and HERCEPTIN treatment: Overexpression of Her2/neu oncogene is observed in approximately 25% of biopsy samples from women with breast cancer and is associated with a poor prognosis. Trastuzumab (HERCEPTIN) is a humanized monoclonal antibody that is directed against the extracellular domain (ECD) of the Her2/neu receptor and inhibits the proliferation of human breast cancer cells overexpressing this receptor (see Esteve FJ 2004, The Oncologist 9(Suppl 3):pp4-9 for a recent review). Protein expression of Her-2/neu on breast cancer cells can easily reach levels of 500,000 molecules or more per cell as is the case of the Her-2/neu overexpressing human breast cancer cell line called SK-BR-3. Current tests for Her2/neu rely on testing tissue sections of the patients biopsy for overexpression of the protein or for gene amplification. In those women with gene amplification or at least 2+ immunostaining for the protein, significant responses have been demonstrated with single agent trastuzumab or with trastuzumab in combination with a chemotherapeutic such as paclitaxel. Other agents which can target Her-2/neu are in development and have a similar applicability as HERCEPTIN for this invention. These include but are not limited to: OMNITARG (pertuzumab) being developed by Genentech; GW-572016 being developed by GlaxoSmithKline (Xia et al., 2004, Oncogene 23:646-653); CP-654577 being developed by Pfizer (Barbacci et al., 2003, Cancer Res 63:4450-4459); HKI-272 being developed by Wyeth (Rabindran et al., 2004, Cancer Res 2004, 64:3958-65). Other anticancer agents that include Her-2/neu among their specificity are described in Janmaat and Giaccone, 2003 (The Oncologist 8:576-86). Besides breast cancer, Her-2/neu is overexpressed on other carcinoma cells including ovarian carcinoma.

ECL: Electrochemiluminescence is a process which uses labels designed to emit light when electrochemically stimulated (for a review on ECL see Yang et al., 1994, Biotechnology 12:193-194; also see Blackburn et al., 1991, Clin Chem 37:1534-1539). These labels, together with the appropriate instrumentation (such as developed by BioVeris) provide for a highly sensitive method of detecting a variety of biological molecules such as proteins, mRNA, and DNA. Martin et al., (2003, US patent #6,524,865) describes an ECL-based enzyme immunoassay; however application to the detection of proteins such as Her-2/neu on circulating cancer cells in blood is not described.

ECL and whole eukaryotic cells:
ECL methods using whole eukaryotic cells have been disclosed by Chinn et al (US patent 6,300,143 B1). However the method by Chinn et al. is for measuring binding affinity of cell surface antibodies rather than the determination of the relative number of receptor molecules on the cell surface. Also the method by Chinn begins with a purified cell population of a large number of cells (167,000 cells per ml) as opposed to first isolating the selected cell population which can be in small numbers (typically 1 to 200 cells per ml; Hayes et al., 2002) from an impure mixture found in whole blood.

Assays for Her-2/neu or related molecules:
The current US Food and Drug Administration (FDA)-approved assays for selecting patients for treatment with HERCEPTIN (trastuzumab) are (1) immunohistochemistry for Her-2/neu protein overexpression (HERCEPTEST by DAKO) and (2) the FISH (fluorescence in-situ hybridization) assay for amplification of the Her-2/neu gene (PATHVISION kit by VYSIS). These tests have been shown to be predictive of the patient response and benefit to HERCEPTIN (Fornier M, et al., 2002. HER2 testing and correlation with efficacy of trastuzumab therapy. Oncology 16:1340-58). However, both of these assays have the following four limitations:
   1) These assays require biopsy tissue. Not all patients will have such archived material readily available for testing. In such cases, the only option would be to obtain a tumor biopsy. An assay of a sample of blood would be much more convenient, easier to perform and has the potential to provide a quicker answer.
   2) Quite often, biopsy samples taken at the time of first diagnosis of breast cancer are used years later when the patient has had a recurrence of disease. Therefore, using assays that test old tissue material, the patient's tumor status to Her-2/neu is being determined at a point in time that can be many years prior to making the clinical decision on whether or not to treat the patient with HERCEPTIN. The data in the literature suggest that a significant number of women (11 of 26 tested) with negative results for Her2/neu in their primary tumor biopsy go on to develop Her2/neu positivity on their circulating cancer cells (Hayes DF, et al., Int J Oncol 21:1111-7; Meng S et al., 2004 Proc Natl Acad Sci USA 101:9393-98) and would thus be candidates for HERCEPTIN treatment. An assay of their Her-2/neu status on such circulating breast cancer cells from a blood sample would afford a more practical and a quicker determination of their current Her-2/neu status.
   3) Due to the subjectivity of the scoring in these assays, significant amounts of disconcordance have been recently shown for results from local laboratory versus central laboratories for both types of assays and can give rise to as high as 25% false positive rate found in some local laboratories (Fornier et al., 2002). Using the more common immunohistochemistry test, there is an estimated 14 to 17% false negative rate as compared to FISH (Seidman et al., 2001, J Clin Oncol 19:2587-95; Fornier et al., 2002). A false negative rate of 14 to 17% using immunohistochemistry would indicate that thousands of such women per year in the USA would be additional candidates for HERCEPTIN therapy.
   4) Finally, these methods are slow requiring (a) tissue blocks to be retrieved from storage at the site where they were taken, (b) sections to be cut, (c) extensive processing, and then most often, (d) time consuming and subjective scoring by trained personnel. These manual analyses, which are commonly used to indicate the degree of cell staining by immunohistochemistry or to indicate the number of positive FISH loci required for scoring the samples, are cumbersome A faster assay to provide quicker feedback to the treating physician would be desirable. In addition, FISH is-very costly and not widely available (Fornier et al., 2002).

Koski, 1998 (US patent 5,783,404) describes immunohistochemical assays of breast cancer cells and breast cancer tissue for the Her-2/neu protein using monoclonal antibodies that recognize a denatured epitope of a specific portion of the Her-2/neu protein. However these assays are in the setting of a pure population of breast cancer cells or, as in the breast cancer tissue, a very high percentage of breast cancer cells. Koski does not address the issue of detecting small numbers of Her-2/neu-expressing breast cancer cells in a complex matrix such as blood.

Slamon et al, 1990 (US patent 4,968,603) describes an assay for amplification of the Her-2/neu gene and uses in screening patients. No examples are given of an assay for detecting the Her-2/neu protein. Futhermore, this patent does not address the issue of detecting small numbers of Her-2/neu-expressing breast cancer cell in a complex matrix such as blood. Application toward identifying patients for treatment of breast cancer patients with a monoclonal antibody against Her-2/neu protein such as HERCEPTIN is not indicated.

Carney et al., 1995 (US patent 5, 401,638) describes an immunoassay of human serum or plasma for the detection of a neu related protein p100 consisting of a truncated version of the full-length Her-2/neu protein consisting of the extracellular domain (ECD) of the human neu gene product. This Carney patent does not address detection of cell-associated or full-length Her-2/neu protein, especially in a complex mixture such as whole blood. A commercially based assay (from ONCOGENE) based on Carney has a sensitivity (1.5 ng of truncated Her-2/neu per mL of serum) that is too low to be used for detecting the potentially small numbers of Her-2/neu protein (pg amounts) that can be detected and is required for detection in accordance with the instant invention. Assays, as in this invention, for cell-associated Her-2/neu are distinctly advantageous since immunohistochemistry of patient biopsy tissue has demonstrated a predictability of cell-associated expression-for-response to HER CEPTIN- treatment. - A recent report by-Burstein et al. (2003, J Clin Oncol 21:2889-2895) indicates the limited usefulness of a serum assay for truncated p100 protein (HER2 ECD). Burstein et al report that "Neither the baseline level of HER2 ECD nor a decrease in HER2 ECD with therapy predicted clinical response to trastuzumab after one cycle." In a study of breast cancer patients treated with HERCEPTIN, Cobleigh et al (1999, J Clin Oncal 9:2639) reached a similar conclusion that there was no significant correlations demonstrable between serum ECD levels and HERCEPTIN response status.

Carney et al., 1997 (US patent 5,604,107) describe an immunoassay for detecting the full-length p185 Her-2/neu protein in cell lysates; however a commercially based ELISA assay based on Carney has a sensitivity that is orders of magnitude too low to be used for detecting the small numbers of Her-2/neu protein (pg amounts) required for the application described here. As noted above, the sensitivity of this assay for quantification of Her-2/neu proteins is reported as 1.5 ng of truncated Her-2/neu per mL of serum ; it would be presumed to be much worse in a more complex mixture such as whole blood. Furthermore, Carney et al. does not address a Her-2/neu assay using whole blood. Instead the level of a truncated Her-2/neu called p100 is detected in the plasma component of blood rather than the cell-associated component. The rationale for preferring an assay measuring cell-associated full-length protein as in this invention rather than an assay measuring plasma levels of the truncated protein are discussed above. Also Carney et al. does not indicate use of whole cells but rather the use of a cell lysate.

Hudziak et al.,1998 (US patent 5,720,937) claims an in vivo assay by exposing cells *within the body* of a mammal to a monoclonal antibody to determine overexpression of the Her-2/neu protein. However, methods of assaying tumor cells outside the body are not described.

Isolation of circulating cancer cells in blood:
Terstappen et al., 2002 (US patent 6,365,362) describes use of immunomagnetic beads with antibodies against epithelial cell adhesion molecule (EpCAM) for the isolation of breast cancer cells from patient blood samples. The Terstappen assay combines immunomagnetic enrichment with flow cytometric and immunocytometric analysis. However, flow cytometry and immunocytometry suffer from being cumbersome and time-consuming techniques.

Consequently, there is a need in medical practice for a rapid and convenient assay that is sensitive enough to use with whole blood samples to identify those women with breast cancer who have overexpression of Her-2/neu protein on circulating breast cancer cells and who therefore are likely to benefit from Her-2/neu-targeting therapy, e.g. trastuzumab.

WO 03/065042 describes methods and reagents for the isolation of circulating cancer cells.

WO 041008099 describes methods for identifying tumours that are responsive to treatment with anti-ErbB2 antibodies.

### SUMMARY OF THE INVENTION

This invention provides a method of detecting the expression of Her-2/neu protein on circulating cancer cells in a blood sample comprising isolating the cancer cells from the blood sample, lysing the isolated cancer cells to yield a cell lysate followed performing on the isolated cancer cells an immunoassay capable of detecting cancer cell-associated Her-2/neu, in which a positive immunoassay result indicates the presence of Her-2/neu on the cancer cells; wherein the cancer cells are isolated by contacting the blood with immunomagnetic beads capable of binding selectively to the cancer cells; wherein the immunoassay uses the detection technique of electrochemiluminescence; wherein the immunoassay uses two antibodies that binds selectively to Her-2/neu. The assay in accordance with this invention has a sensitivity defined by: a) being capable of detecting cancer cell-associated Her-2/neu at a level of between one-tenth picogram and twenty picograms of Her-2/neu per milliliter of the blood sample; or b) being capable of detecting Her-2/neu from SK-BR-3 breast cancer cells when spiked into blood at a concentration of less then or equal to 100 SK-BR-3 cells per milliliter of blood.

This invention provides a method of identifying a cancer patient likely to benefit from treatment with an anticancer agent that targets Her-2/neu, comprising the detection method described above. When used to identify such patients, the cancer cell-containing blood sample is a sample from the patient. The specification describes treating an anticancer agent for use in a cancer patients identified according to the above method which method comprises administering a Her-2/neu. targeting anticancer agent to the patient.

### BRIEF DESCRlPTION OFTHE FIGURES

Figure 1: Detection of recombinant Her-2/neu (4, 16 and 64 pg/well) by ECL immunossay.
Figure 2. Detection of Her-2/neu from extracts of SK-BR-3 human breast carcinoma cells (10,30 and 100 cells lysate material per well). The cell lysis reagent used was the Sigma Lysis Buffer.
Figure 3. Detection of Her-2/neu from extracts of SK-BR-3 human breast carcinoma cells (10, 30 and 100 cells lysate material per well). The cell lysis reagent used was the Pierce Lysis Buffer.
Figure 4. Comparison of the BCL signal for the immunoassay detection of Her-2/neu in lysates from SK-BR-3 breast cancer cells (positive control for Her-2/neu overexpression) versus MDA-MB-468 breast cancer cells (negative for Her-2/neu oyerexpression). For each cell line, lysate material from 10, 30 and 100 cells/well were used.
Figure 5. Comparison of the ECL signal for the immunoassay detection of Her-2/neu in lysates from SK-BR-3 breast cancer cells (positive control for Her-2/neu overexpression) using lysate material from 0.9, 3 and 10 cells/well versus MDA-MB-468 breast cancer cells (negative for Her-2/neu overexpression) using lysate material from 0.9, 3, 10 and 100 cells/well.
Figure 6. Lack of interference by lysates of PBMCs (from 100 to 10,000 cells/well) for the immunoassay detection of Her-2/neu in lysates from SK-BR-3 breast cancer cells (positive control for Her-2/neu overexpression) using lysate material from 1, 3 and 10 SK-BR-3 cells/well.

### DETAILED DESCRIPTION OF THE INVENTION

As used herein the transitional term "comprising" is open-ended. A claim utilizing this term can contain elements in addition to those recited in such claim. Thus, for example, the claims can read on methods that also include other steps not specifically recited therein, as long as the recited element or their equivalent are present.

This invention provides methods sensitive enough for quantifying the levels of Her-2/neu protein on circulating breast cancer cells in blood samples and provides methods for identifying those women with breast cancer who are likely to benefit from therapy using HERCEPTIN or another agent targeted to Her-2/neu A convenient, highly sensitive and rapid means to test blood samples to identify additional patients who would benefit from HERCEPTIN therapy would be an important advance in the breast cancer treatment field. As indicated below, the rapid and highly sensitive immunological assay used to detect Her-2/neu protein on the surface of circulating breast cancer cells is using electochemiluminescence (ECL)-detection.

This invention is based on combining the high specificity of procedures used to isolate circulating carcinoma cells from blood with the high sensitivity of certain immunologically based assays such as ECL. Preferably and advantageously, immunomagnetic beads are used for both aspects of the invention, thus serving a novel dual function: Immunomagnetic beads are used for isolating and purifying the circulating cancer cells from blood and either different beads or these same beads can then be used as a support phase for performing the ECL allowing a magnet to capture them and concentrate target antigen along with tagged antibody (e.g., ruthenium tag labeled antibody).

In embodiments of the detection method of this invention the sensitivity level in a) above is between one and twenty picograms, between one and ten picograms, or between one and five picograms, of Her-2/neu per milliliter of the blood sample. In another embodiment of the detection method of this invention the sensitivity level in b) above is being capable of detecting Her-2/neu from SK-BR-3 breast cancer cells when spiked into blood at a concentration of less than or equal to ten SK-BR-3 cells per milliliter of blood. In further embodiments the sensitivity level in b) above is being capable of detecting Her-2/neu from SK-BR-3 breast cancer cells when spiked into blood at a concentration of less than or equal to three SK-BR-3 cells per milliliter of blood, and even less than or equal to one SK-BR-3 cells per milliliter of blood.

A sample (usually in the range of approximately 8 to 20 ml) of blood from a patient with cancer, especially breast cancer, is taken. Steps include as detailed below:
1. Removal of red blood cells
2. Optional negative selection to futher deplete normal leukocytes. The preferred embodiment includes this step.
3. Positive selection for circulating carcinoma cells
4. Detection wand quantification of the Her-2/neu protein from circulating carcinoma cells

### 1. Removal of red blood cells.

A variety of methods are available to remove red cells including but not limited to separation based on density (such as collection of blood directly into the BECTON DICKINSON BD Vacutainer CPT tubes) followed by centrifugation) and commercial lysing buffers such as PURESCRIPT RBC lysis buffer (GENTRA, Minneapolis), FACS lysing solution (BDIS), IMMUNOLYSE (COULTER), OPTILYSE B (IMMUNOTECH), and ACK lysing buffer (BIOSOURCE, Rockville, MD).

A preferred method uses the BD Vacutainer CPT tubes with anticoagulant (EDTA or citrate). These tubes contains a material that upon correct centrifugation (1,100xg for 10 minutes, swing-out bucket rotor) allows for elimination of red blood cells and neutrophils. After centrifugation, the bottom of the tube contains a cell pellet of erythrocytes (red blood cells) and neutrophils. Above the cell pellet is a gel barrier and above the gel barrier are tumor cells, lymphocytes and monocytes as a band at the bottom of the plasma. The tumor cells, lymphocytes and monocytes can then be readily collected from the top above the gel barrier. This method is preferred as it removes not only the red blood cells but also the neutrophils.

### 2. Negative selection to further deplete normal leukocytes.

A preferred embodiment of this invention uses of negative selection step for isolation of tumor cells. Negative selection allows for further depletion of leukocytes especially the lymphocytes and monocytes. This step comprises the use of antibodies that are bispecific for both leukocyte antigens, especially CD45, the common leukocyte antigen, and for a red blood cell antigen such as glycophorin A. A commercially available cocktail of such bispecific antibodies is available from STEMCELL TECHNOLOGIES (Rosettesep Catalog #15127 and #15167). This cocktail includes bispecific antibodies against glycophorin A and against a variety of cell surface antigens on human hematopoietic cells (CD2, CD16, CD19, CD36, CD38, CD45, CD66b). One or more of these bispecific antibodies are added to the BD Vacutainer CPT tubes before blood collection. In the preferred embodiment, the cocktail ofbispecific antibodies against more than one leukocyte-associated CD molecule is used. When the blood is introduced into the CPT vacutainer tube, the bispecific antibodies form immunorosettes each consisting of leukocytes plus many red blood cells. These immunorosettes have a density approximately that of red blood cells and when centrifuged are found in the red blood cell pellet, thus further removing leukocytes from the tumor cell fraction found above the cell pellet and gel barrier. The fraction with the tumor cells in plasma is collected for further processing.

### 3. Positive selection for circulating carcinoma cells.

Circulating carcinoma cells are isolated using immunomagnetic beads. Other methods of isolation of circulating cancer cells include filtration (Vona G et al., 2000, Am J Pathol. 2000 156:57-63). The immunomagnetic beads have antibodies against antigens found selectively on the surface of carcinoma cells such as Her2/neu . The immunomagnetic beads may be of various sizes (50 microns to less than 200 nm) and include DYNAL beads (> 1.5 microns to about 50 microns) with antibodies against Her2/neu. In the preferred embodiment nanoparticle beads are used as it will allow for faster and more efficient binding of tumor cells to the beads. In an embodiment of the invention, EasySep^{™} human EpCAM positive selection cocktail and EasySep^{™} Magnetic nanoparticles (STEMCELL TECHNOLOGIES) are added to the fraction with the tumor cells in plasma from the previous step. A magnet is then used to separate tumor cells from the rest of the material and the tumor cells are washed with an aqueous solution. Purified tumor cells are then ready for detection of antigens in the next step.

### 4. Detection and quantification of the Her-2/neu protein from circulating carcinoma cells.

Detection of Her-2/neu can then be accomplished by use of two antibodies such as a monoclonal antibody . (mAb) such as HERCBPTIN or mAb 191924 (R&D systems Catalog number MAB 1129) or a polyclonal antibody against Her-2/neu (e.g., Goat polyclonal antibody catalog number AF1129 from R&D systems) that are linked to a detecting molecule. In the case of electrochemiluminescence (BCL), the detecting molecule is ruthenium. There is abundant literature in the public domain provides amply useful methods for linking ruthenium to antibodies (eg.,, Lee et al., Am J Trop Med Hyg 2001, 65:1-9) followed by ECL detection of antigens on magnetic beads in a solution containing tripropylamine. With application of an electric potential, the rhuthenium label is excited and light is emitted and detected using an ECL detecting instrument (such as the ORIGEN analyzer or a commercially available instrument like the M-Series® 384 from BIOVERIS Corporation, Gaithersburg. MD)

The immunoassay utilized in accordance with this invention can be either polyclonal or monoclonal antibodies against Her-2/neu. Preferably the monoclonal antibody is a humanized mouse monoclonal antibody, e.g. trastuzumab. Trastuzumab is preferred for the immunoassay methods in accordance with this invention.

For purposes of detection of Her-2/neu, a variety of monoclonal and polyclonal antibodies against Her-2/neu and include antibodies against the domain and against the cytoplasmic domain are commercially available from such sources as R&D Systems (Minneapolis, MN Biosource (Camarillo, CA) and BD Biosciences, San Diego, CA). Rabbit polyclonal antibodies are also available from LABVISION Corp, Fremont. CA; such as neu Ab-21) and from UPSTATE CELL SIGNALING SOLUTIONS (Lake Placid, NY; such as Catalog number 06-562). A goat polyclonal antibody against the domain from Her-2/neu is available from R&D systems (catalog number AF1129). A goat polyclonal antibody against full-length recombinant Her-2/neu is available from EXALPHA BIOLOGICS (Rosedale, MA; catalog number M100P). Such polyclonal antibody against full-length Her-2/neu would be expected to be able to bind to and cytoplasmic domains of Her-2/neu and not to be specific for the extracellular domain. Monoclonal antibodies are available against both the extracellular domain (e.g., R&D Systems Catalog number MAB1129) and the cytoplasmic domain. (e.g., LABVISION neuAB-8) including against the C-terminal peptide (e.g., LABVISION neuAB-15). Monoclonal antibodies against Her-2/neu are also disclosed in Hudziak et al (1997, US patent 5,677,171). One improved embodiment uses HERCEPTIN since binding with this antibody is best able to predict binding of HERCEPTIN as a treatment in the patient. Another advantageous embodiment uses a rabbit polyclonal antibody or a cocktail of antibodies binding to many epitopes on the Her-2/neu protein allows for higher sensitivity.

The isolated cancer cells are lysed prior to the immunoassay and the immunoassay is performed on the cell lysate. In this case the immunoassay can utilize antibodies that bind selectively either to the extracellular or cytoplasmic domain of Her-2/neu. In a more specific embodiment of this invention the immunoassay uses one or two antibodies that bind selectively to the cytoplasmic domain of Her-2/neu.

The immunoassay of this invention is more rapid and has a significantly greater sensitivity than any previously developed immunoassay for Her-2/neu. The immunoassay of this invention is capable of detecting Her-2/neu expression from 100 or less of SK-BR-3 breast cancer cells added per ml of blood from a human volunteer without cancer. The immunoassay of this invention is capable of detecting cancer cell-associated Her-2/neu at a level of twenty picograms or less of Her-2/neu per milliliter of a blood sample

Besides electrochemiluminesence, other immunoassays that can yield a high sensitivity required for this application include, but are not limited to:
a) Chemiluminescence such as described by Liu Y et al., 2003 (J Food Protection 66:512-7).
b) Fluorogenic-chemiluminescence (FCL) as described by Yu H et al., 2000 (Biosens Bioclectron 14:829-40)
c) Fluorescence polarization immunoassay (see Howanitz JH, 1988 Arch Pathol Lab Med 112:775-9)
d) Time-resolved fluorescence immunoassay (Butcher H et al., 2003, J Immunol Methods 272:247-56; Soukka et al., 2001, Clin Chem 47:1269-78; Hovanitz JH, 1988 Arch Pathol Lab Med 112:775-9)

In a preferred embodiment, the relative quantity of breast cancer cells used in the assay is estimated. This allows for a ratio of total Her-2/neu protein per cell to be obtained and can be compared to control standards of breast cancer cells with high, moderate, and low levels of Her-2/neu protein per cell. This is the preferred embodiment since it eliminates a false positive situation in which there are many circulating breast cancer cells that have a low level of Her-2/neu protein expression that may give a signal that mimics that obtained from a small number of breast cancer cells with a high level expression. In this embodiment, a variety of approaches can be used to estimate relative cell numbers including flow cytometic analysis, quantification of total DNA or DNA related antigens such as histones from lysed cells (there is 6 pg DNA per diploid cell), and turbidity or absorbance measurements.

Due to its sensitivity the method according to this invention for identifying patients likely to benefit from treatment with an anticancer agent that targets Her-2/neu can be fruitfully applied to patients from whom a tumor biopsy tissue had been previously determined (e.g. by immunohistochemistry or FISH analysis) to be negative for Her-2/neu expression by a tissue assay for Her-2/neu.

The invention will be better understood by reference to the following examples, which illustrate but do not limit the invention described herein.

### EXAMPLES

### Example 1

A patient with metastatic breast cancer comes into the office and a blood sample (8 to 40 mL) is withdrawn directly into BD Vacutainer CPT tubes containing an anticoagulant such as citrate as well as an added negative selection product: ROSETTESEP (from STEMCELL TECHNOLOGIES) containing bispecific antibodies toward erythrocytes antigens as well as toward leukocyte surface antigens. The material is centrifuged for 20 minutes at 1500 to 1800 RCF (relative centrifugal force). The cell layer above the gel barrier is removed. EasySep^{™} human EpCAM positive selection cocktail and EasySep^{™} Magnetic nanoparticles (StemCell Technologies) are added and the tumor cells isolated and washed using a magnetic field. Ruthenium-labeled polyclonal antibody against Her-2/neu is added along with a solution of tripropylamine to the tumor cells attached to the magnetic beads bound to an electrode. Routine methods of ruthenium labeling the antibody are described in the art such as Lee et al., Am J Trop Med Hyg 2001, 65:1-9. An electric current is applied and electrochemiluminescence (ECL) is detected using an ECL detection device such as one commercially available (BIOVERIS Corporation). Within these instruments is a photomultiplier tube (PMT) placed just above the working electrode for efficient light capture. Under the working electrode, a magnet is in place for capturing the beads coated with the target antigen. The signal is proportional to the amount of Her-2/neu found bound on the surface of the circulating tumor cells.

### Example 2

Methods identical to that used in example 1 are provided except that a monoclonal antibody rather than a polyclonal antibody to Her-2/Neu is used for detection.

### Example 3

Methods identical to that used in examples 1-2, except that isolated breast cancer cells are lysed either before addition of an antibody against Her-2/neu linked to a magnetic bead. Lysis can be achieved with any number of cell lysis reagents described in the art such as, but not limited to Lysis Buffer A [1% NP-40, 20 mM Tris (pH 8.0), 137 mM NaCI, 10% glycerol, 2 mM EDTA, 1 mM sodium orthovanadate, 10 ug/mL Aprotinin, 10 Ug/mL Leupeptin] and RIPA buffer (Papetti and Herman, 2001, Am J Pathology 159:165-178). In this sandwich type ECL (see Yang et al., 1994, for an illustration of a 'sandwich' immunoassay using ECL), two sets of antibodies against Her-2/neu are used: one antibody is biotinylated and attached to strepavidin-coated magnetic beads while the second antibody is ruthenium-labeled.

### Example 4

Methods identical to that used in examples 1 through 3 are provided except that the patient had a prior negative result for Her2/neu based on analysis of her primary tumor or does not have tumor tissue readily available for analysis.

### Example 5

Methods as that used in examples 1 through 4 are provided with the addition of a step to quantify the relative number of breast cancer cells are used in the analysis An ELISA to quantify cells by DNA content (there are 6 pg of DNA per diploid nucleus (www.gentra.com/calcularing.asp) has described by Friis et al (2003; APMIS 111:658-68) but lacks the sensitivity required for this invention. A higher sensitivity assay required for the detection of the small number of cells required in this invention is achieved using a highly-sensitive immunoassay, e.g., by using ECL and the same instrument used to quantify Her-2/neu.

In order to quantity cells by ECL, they are first lysed (e.g., with lysis buffers such as, but not limited to, Lysis Buffer A as detailed above) and then two different antibodies directed against double stranded DNA (e.g., Mouse monoclonal antibody HYB 33-01 available from STATENS SERUM INSTITUT (Copenhagen, Denmark); Mouse monoclonal antibody MAB3032 available from CHEMICON (Temecula, CA, USA); Mouse Monoclonal Antibody catalog number DNA11-M from ALPHA DIAGNOSTICS INTERNATIONAL (San Antonio, TX, USA) are added; one has been labeled with ruthenium (routine methods of ruthenium labeling the antibody are described in the art such as Lee et al, Am J Trop Med Hyg 2001, 65:1-9) and the other labeled with biotin for attachment to strepavidin-coated magnetic beads. Quantification of antigen [in this case dsDNA (double-stranded DNA)] is achieved by ECL with a solution of tripropylamine and the magnetic boads bound to an electrode by use of a magnetic field. An electric current is applied and electrochemiluminescence (ECL) is detected using an ECL detection device such as one commercially available (BIOVERIS Corporation). Within these instruments is a photomultiplier tube (PMT) placed just above the working electrode for efficient light capture. The signal is proportional to the amount of dsDNA and, therefore, is proportional to the cell number. This then allows for a ratio of Her-2/meu per cell number to be obtained. This ratio, rather than an absolute number of tumor cell-associated Her-2/neu per ml blood, is advantageous in this invention for determining the patient's sensitivity toward HERCEPTIN treatment.

### Example 6

A patient with a level of Her-2/neu above control samples as indicated in Examples 1-5 is deemed to have tumor cells positive for Her-2/neu and then treated with a regimen containing a monoclonal antibody against Her2/neu such as HERCEPTIN. A preferred treatment consists of HERCEPTIN at an initial loading dose of 4 mg/kg administered as a 90 minute infusion with a weekly maintenance dose of 2 mg/kg as a 30 minute infusion.

### Example 7

In this example, purified recombinant Her-2/neu (extracellular domain) was used as a standard to examine the sensitivity of a sandwich immunoassay using electrochemiluminescence.

Four different PBS assay buffers were prepared:
- Assay Buffer 1: 0.5% Tween-20 and 0.5% bovine serum albumin (BSA) in PBS (phosphate buffered saline)
- Assay Buffer 2: 1.0% Tween-20 and 0.5% BSA in PBS
- Assay Buffer 3: 0.5% Tween-20 and 1.0% BSA in PBS
- Assay Buffer 4: 1.0% Tween-20 and 1.0% BSA in PBS

Her-2/neu standard (recombinant Her-2/neu extracellular domain) was obtained from DakoCytomation (Carpinteria, CA 93013 USA; Product EL541). Goat anti-human Her-2/neu polyclonal antibody was obtained in both biotinylated and non-biotinylated forms (catalog numbers BAF1129 and AF1129, respectively) from R&D Systems, Inc. (Minneapolis, MN 55413 USA) as was the monoclonal antibody MAB1129 (R&D Systems catalog number 191924). The polyclonal antibody AF1129 and monoclonal antibody MAB1129 were ruthenium labeled ("TAG-labeled") as follows:
- 1.5 µg/µl ruthenium label (BV-TAG-NHS Ester, Catalog # 110034; BioVeris Corporation, Gaithersburg, MD, USA) was prepared in DMSO.
- For 500 µl of monoclonal antibody (protein concentration of 1 mg/ml), 18.8 µl BV-TAG-NHS was added and for 200 µl of polyclonal antibody (protein concentration of 0.5 mg/ml), 3.8 µl BV-TAG-NHS was added. In each case, the solution was incubated for one hour and the reaction stopped by the addition of 20µl of 2M glycine.
- Uncoupled BV-TAG-NHS Ester in each reaction mixture was removed using a PD-10 gel filtration column, pre-equilibrated with PBS (including 0.08% sodium azide), which was also used for elution. For each antibody, the protein concentration in each fraction was determined by protein assay and the fractions with high protein content were used in subsequent examples.

The ruthenium-labeled polyclonal antibody AF1129 and the biotinylated polyclonal antibody BAF1129 are referred hereafter in this example and subsequent examples as "TAG-pAb" and "Biotin-pAb". The ruthenium-labeled monoclonal antibody MAB1129 is referred hereafter in this example as "TAG-mAb".

An electrochemiluminsence assay was performed as follows:
- Sequentially, Her-2/neu standards in 25µl/well and then 50 µl/well of a mixture of TAG-Ab and Biotin-Ab (e.g., at a concentration of 1 µg/ml each; diluted into the 4 PBS assay buffers) were added to wells of a 96-well U-bottom polypropylene plate and incubated at room temperature with constant shaking (e.g., for 2 hours).
- 10µg of magnetic streptavidin beads (e.g., Dynabeads M-280 Streptavidin, Catalog #110028, BioVeris, Corporation, Gaithersburg, MD) in 25µl was added to each well and incubated with constant shaking (e.g., for 30 minutes).
- PBS assay buffer was added to each well to make a final volume of 250 µl per well. The amount of the analyte (recombinant Her-2/neu extracellular domain) in this assay was varied from 16 to 1600 pg per well. Control wells without analyte were also included. All conditions were tested in at least duplicate wells. The 96 well plate was then analyzed for electrochemiluminescence using the M8 M-Series® Analyzer (Catalog Number 310800, BioVeris, Corporation, Gaithersburg, MD).

Results showed that all tested levels of recombinant Her-2/neu extracellular domain (16, 160 and 1600 pg/well) were detectable and above baseline using all four different assay buffers using the sandwich immunoassay with TAG-pAb and Biotin-pAb (Table 1). Recombinant Her-2/neu extracellular domain was also detectable using the sandwich immunoassay with TAG-mAb and Biotin-pAb (Table 2).

**Table 1. Electrochemiluminescence (ECL) detection of recombinant Her-2/neu by immunoassay using ruthenium-labeled polyclonal (TAG-pAb) and biotinylated polyclonal antibody (Biotin-pAb).**

| | Mean ECL Signal (above backgound)* | | | |
|---|---|---|---|---|
| Her-2/neu (pg/well) | Using Assay Buffer-1 | Using Assay Buffer-2 | Using Assay Buffer-3 | Using Assay Buffer-4 |
| 16 | 146 | 88 | 89 | 93 |
| 160 | 998 | 884 | 888 | 850 |
| 1600 | 9690 | 9466 | 8553 | 8750 |

| | | | | |
|---|---|---|---|---|
| *Mean ECL signal above the mean signal from control wells with no antigen. | | | | |

**Table 2. Electrochemiluminescence (ECL) detection of recombinant Her-2/neu by immunoassay using ruthenium-labeled monoclonal (TAG-mAb) and biotinylated polyclonal antibody (Biotin-pAb).**

| | Mean ECL Signal (above background)* | | | |
|---|---|---|---|---|
| Her-2/neu (pg/well) | Using Assay Buffer-1 | Using Assay Buffer-2 | Using Assay Buffer-3 | Using Assay Buffer-4 |
| 16 | ** | ** | * | 8 |
| 160 | ** | ** | 12 | 39 |
| 1600 | 38 | 34 | 57 | 96 |

| | | | | |
|---|---|---|---|---|
| * Mean ECL signal above the mean signal from control wells with no antigen. ** Signal not above the mean signal from control wells with no antigen. | | | | |

### Example 8

Methods as that used in example 7 were used except that:
- The PBS assay buffer used throughout this example was PBS assay buffer 1.
- The only ruthenium labeled antibody used was Tag-pAb. The concentration of Tag-pAb added was 2 µg/ml in 50 µl instead of 1 µg/ml.
- The amount of recombinant Her-2/neu (extracellular domain) was varied from 4 to 64 pg/ml.

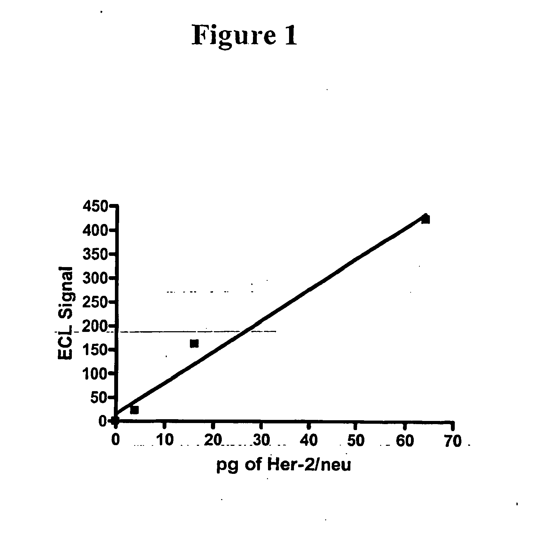

Results showed that all tested levels of recombinant Her-2/neu extracellular (4, 16, and 64 pg/well) were clearly detectable and above baseline (see Figure 1).

### Example 9

Methods as that used in example 8 except that:
- The amount of recombinant Her-2/neu (extracellular domain) was varied from 16 pg/well to 4096 pg/well.

The ability to detect Her-2/neu was tested in the presence or absence of 0.02 µl/well of cell lysis buffer. Pierce Lysis Buffer [M-PER® Extraction Reagent (Product number 78501 from Pierce Biotechnology, Inc., Rockford, IL)] and Sigma Lysis Buffer [Sigma CelLytic^{™}-M (Sigma Product Nubmer C 2978, Sigma-Aldrich, Inc., St. Louis, MO 63103)] were tested in separate wells.

The result are presented in Table 3. All amounts of Her-2/neu in this experiment including the lowest amount of 16 pg/lwell were detectable and above baseline (Table 3).. This finding was observed in the presence or absence of each cell lysis buffer (Pierce Lysis Buffer or Sigma Lysis Buffer).

**Table 3. ECL immunoassay detection of recombinant Her-2/neu.**

| | Mean ECL Signal (above background) | | |
|---|---|---|---|
| Her-2/neu (pg/well) | Using Assay Buffer-1 | Using Assay Buffer-1 with Pierce Lysis Buffer | Using Assay Buffer-1 with Sigma Lysis Buffer |
| 0 | 0 | 0 | 0 |
| 16 | 298 | 226 | 274 |
| 64 | 480 | 436 | 482 |
| 256 | 1203 | 1043 | 1174 |
| 1024 | 4341 | 3998 | 4055 |
| 4096 | 13976 | 15046 | 14097 |

### Example 10.

Immunoassay methods were as that used in example 8 except that:
- Cell extracts from SK-BR-3 breast carcinoma cells were analyzed.

SK-BR-3 cells (from ATCC, Manassas, VA) were grown in 6-well tissue culture plates as per ATCC recommended conditions, washed two times with PBS, and an aliquot counted using a hemacytometer. Lysis of SK-BR-3 cells was performed using either the Pierce Lysis Buffer or Sigma Lysis Buffer. These two lysis buffers are described in Experiment 9 above. In order to lyze SK-BR-3 cells, 200 µl lysis buffer were added per 1 million cells. Cell lysis were performed as per each manufacture's recommendation with the addition of 5 minutes of vigorous vortexing prior to cell debris removal. Cell debris was removed from the cell lysate by centrifugation at 14,000 rpm for 30 minutes in an Eppendorf Centrifuge (Model 5415C). The amount of lysate supernatant per well was varied from that extracted from 10 to 1000 SK-BR-3 cells and analyzed for Her-2/neu using the immunoassay described in Experiment 7 with PBS Assay Buffer 1.

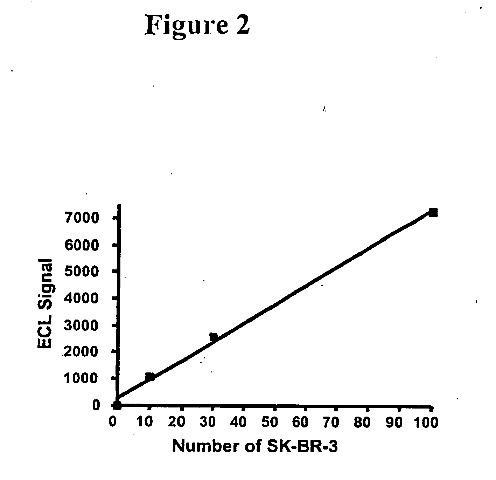

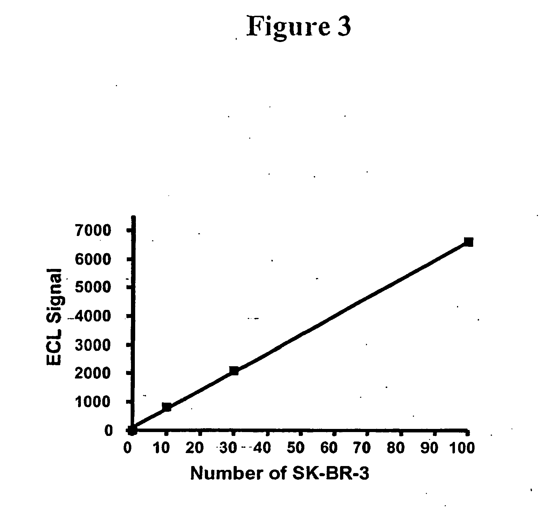

The results from this experiment are presented in Table 4. Her-2/neu was detectable and above baseline from lysates from SK-BR-3 cells in this experiment including those wens using the lowest amount of SK-BR-3 lysate in this experiment (lysate from 10 cells added per well; Table 4). This result was observed regardless of the cell lysis buffer (Pierce Lysis Buffer or Sigma Lysis Buffer) used. Figures 2 and 3 are graphic displays of the results using Sigma Lysis Buffer and Pierce Lysis Buffer, respectively, for lysates from the three lowest amounts of SK-BR-3 cells tested per well (10, 30 and 100 SK-BR-3 cells per well) and demonstrate the linearity of Her-2/neu detection from cells using this immunoassay.

**Table 4. ECL immunoassay detection of Her-2/neu in SK-BR-3 breast cancer cell lysates.**

| | Mean ECL Signal (above background) | |
|---|---|---|
| Lysates from SK-BR-3 Cells (cells/well) | Using Pierce Lysis Buffer | Using Sigma Lysis Buffer |
| 0 | 0 | 0 |
| 10 | 83-1 | 1079 |
| 30 | 2076 | 2574 |
| 100 | 6587 | 7225 |
| 300 | 19402 | 19436 |
| 1000 | 56866 | 54339 |

### Example 11

In this experiment, immunoassay methods were as that used in example 10, except that lysates using Sigma Lysis Buffer from the following cells were examined:
- SK-BR-3 human breast carcinoma cells (high expression of Her-2/neu; Goebel SU, et al., 2002, Cancer Res 62:3702-10); see Example 10 for preparation. This SK-BR-3 cell line is an appropriate positive control for breast cancer expression of Her-2/neu protein.
- MDA-MB-468 human breast carcinoma is an appropriate negative control breast cancer cell line for overexpression of Her-2/neu (Goebel SU, et al., 2002, Cancer Res 62:3702-10). Cell growth and cell lysis using Sigma Cell Lysis Buffer were performed for MDA-MB-468 cells as in Example 10 for SK-BR-3 cells.

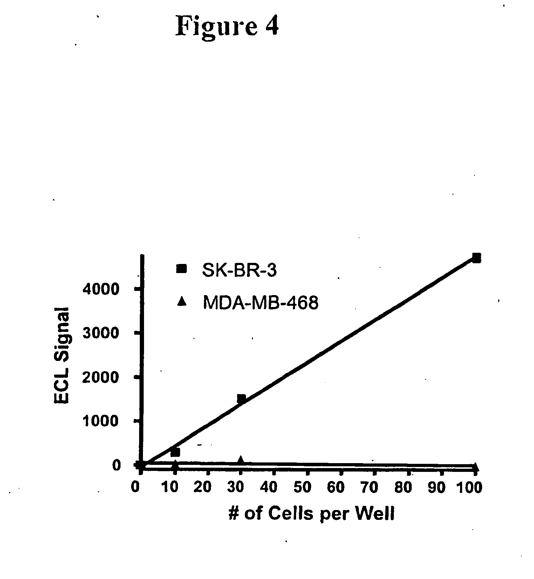

Results in this experiment are presented in Figure 4. The lysate from the SK-BR-3 cells (positive control for Her-2/neu overexpression) gave a much higher signal in the immunoassay for Her-2/neu than the lysate from MDA-MB-468 cells (negative for Her-2/neu overexpression) indicating the specificity of the results for Her-2/neu detection (Figure 4).

### Example 12

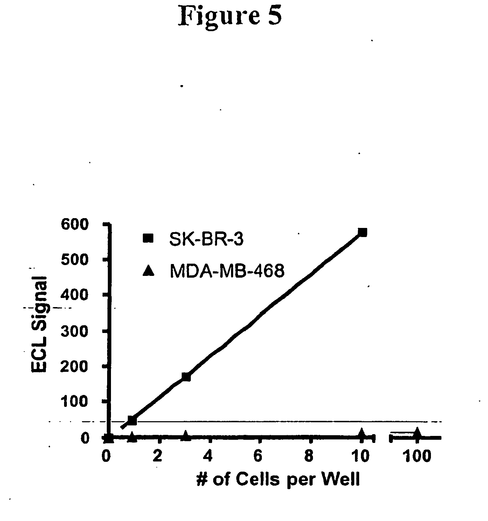

In this experiment, immunoassay methods were as that used in example 11, except that the concentration for both Tag-pAb and Biotin-pAb added in 50 µl was 0.5 µg/ml each instead of µg/ml each. Again the cell lysate from SK-BR-3 cells was compared in terms of Her-2/neu expression to the cell lysate from MDA-MB-468 cells. Results in this experiment are presented in Figure 5. The lysate from the SK-BR-3 cells (positive control for Her-2/neu overexpression) gave a much higher signal in the immunoassay for Her-2/neu than the lysate from MDA-MB-468 cells (negative for Her-2/neu overexpression) indicating the specificity of the results for Her-2/neu detection (Figure 5). Also, Her-2/neu was detectable from lysate material from as little as 0.9 SK-BR-3 cells per well; this gave an ECL signal above background and also above the signal from lysate material from 100 MDA-MB-468 cells (Figure 5).

### Example 13

In this experiment, immunoassay methods were as that used in example 12, except that an additional cell lysate (mouse peripheral blood mononuclear cells, PBMCs) was obtained using the Sigma Lysis Buffer. PMBCs are composed of lymphocyes and monocytes and may co-purify during the initial steps leading to the isolation of circulating breast cancer cells from blood.

Four ml of mouse blood was collected in a 4-ml BECTON DICKINSON BD Vacutainer CPT tube and centrifuged at 3000 rpm for 30 minutes in a Jouan CR412 centrifuge. PBMCs were collected in the cell fraction above the gel and washed 4 times with PBS. A total of 1 million PBMCs were collected from the 4 ml of blood. Cell lysis was performed as for SK-BR-3 cells. Cell debris was removed from the cell lysate by centrifugation at 14,000 rpm for 30 minutes in an Eppendorf Centrifuge (Model 5415C). The supernatant was then used for analysis in this experiment.

Samples for testing were prepared by adding into each well the following:
- The cell lysate from 1 to 10 SK-BR-3 cells in 12.5 µl.
- The cell lysate from 100 to 10,000 PBMCs or control PBS assay buffer 1, again in 12.5 µl.
- 50 µl of a solution containing Tag-pAb with Biotin-pAb (each antibody at 0.5 µg/ml in the 50 µl) was added per well and the 96-well plate was incubated with constant shaking for 2 hours at room temperature.
- 10µg of magnetic streptavidin beads (e.g., Dynabeads M-280 Streptavidin, Catalog #110028, BioVeris, Corporation, Gaithersburg, MD) in 25µl was added to each well and incubated with constant shaking for 30 minutes.
- PBS assay buffer-1 was added to each well to make a final volume of 250 µl per well. All conditions were tested in at least triplicate wells. The 96 well plate was then analyzed for electrochemiluminescence using the M8 M-Series® Analyzer (Catalog Number 310800, BioVeris, Corporation, Gaithersburg, MD).

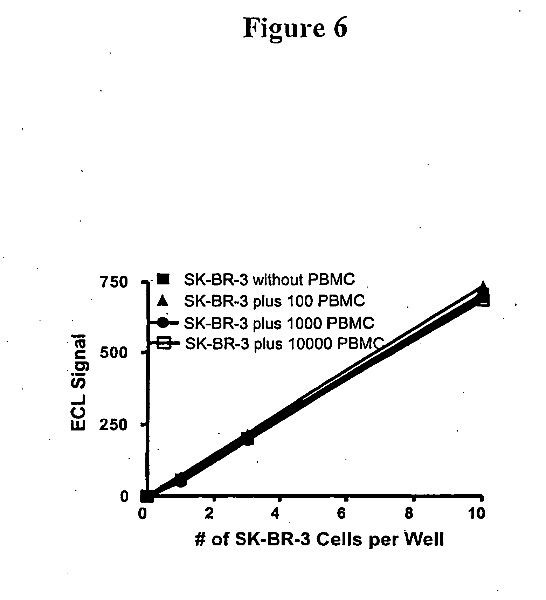

Results in this experiment are presented in Tables 5 and 6 and in Figure 6. Her-2/neu was undetectable from 100, 1000, and 10,000 PBMCs (Table 5). In contrast, Her-2/neu was detectable from even the smallest amount of SK-BR-3 breast cancer cell lysate used (lysate from 1 SK-BR-3 cell per well; see Table 6). Furthermore, the addition of cell lysates from 100, 1000 and even 10,000 PBMCs did not interfere with the detection of Her-2/neu in breast cancer cells (Table 6 and Figure 6). PMBCs, which may co-purify during the initial steps leading to the isolation of circulating breast cancer cells from blood, had undetectable Her-2/neu expression and also did not interfere with the detection of Her-2/neu on SK-BR-3 cells.

**Table 5. Her-2/neu is not detectable on large numbers (e.g, 10,000) of PBMCs as determined by ECL immunoassay.**

| Amount of PBMC Lysate (cells/well) | Mean ECL Signal (above background) |
|---|---|
| 0 | 0 |
| 100 | Negative* |
| 1000 | Negative |
| 10,000 | Negative |

| | |
|---|---|
| • Negative: ECL signal slightly below background level. | |

**Table 6. ECL immunoassay detection of Her-2/neu in SK-BR-3 breast cancer cell lysates in the presence or absence of lysates from PBMCs.**

| | Mean ECL Signal (above background) | | | | |
|---|---|---|---|---|---|
| Lysates from SK-BR-3 Cells (SK-BR-3 cells/well) | Without lysates from PBMCs | With lysates from 100 PBMCs per well | With lysates from 1000 PBMCs per well | With lysates from 3000 PBMCs per well | With lysates from 10,000 PBMCs per well |
| 0 | 0 | 0 | 0 | 0 | 0 |
| 1 | 57 | 68 | 47 | 61 | NT |
| 3 | 206 | 218 | 194 | 192 | 201 |
| 10 | 710 | 733 | 695 | NT | 686 |

| | | | | | |
|---|---|---|---|---|---|
| NT: Not tested. | | | | | |

## Claims

1. A method of detecting the expression of Her-2/neu protein on circulating cancer cells in a blood sample comprising isolating the cancer cells from the blood sample, lysing the isolated cancer cells to yield a cell lysate, followed by performing on the cell lysate an immunoassay capable of detecting cancer cell-associated Her-2/neu, in which a positive immunoassay result indicates the presence of Her-2/neu on the cancer cells;
wherein the cancer cells are isolated by contacting the blood with immunomagnetic beads capable of binding selectively to the cancer cells;
wherein the immunoassay uses the detection technique of electrochemiluminescence;
wherein the immunoassay uses two antibodies that bind selectively to Her-2/neu; and wherein the immunoassay has a sensitivity defined by:
a) being capable of detecting cancer cell-associated Her-2/neu at a level of between one-tenth and twenty picograms of Her-2/neu per milliliter of the blood sample; or
b) being capable of detecting Her-2/neu from SK-BR-3 breast cancer cells when spiked into blood at a concentration of less than or equal to 100 SK-BR-3 cells per milliliter of blood.

2. The method of claim 1, wherein the sensitivity level in a) is between one and twenty picograms of Her-2/neu per milliliter of the blood sample, preferably wherein the sensitivity level in a) is between one and ten picograms of Her-2/neu per milliliter of the blood sample, and more preferably wherein the sensitivity level in a) is between one and five picograms of Her-2/neu per milliliter of the blood sample.

3. The method of claim 1 or claim 2, wherein the sensitivity level in b) is less than or equal to 10 SK-BR-3 cells per milliliter of blood, preferably wherein the sensitivity level in b) is less than or equal to 3 SK-BR-3 cells per milliliter of blood, more preferably wherein the sensitivity level in b) is less than or equal to 1 SK-BR-3 cell per milliliter of blood.

4. The method of any preceding claim, wherein the immunoassay utilizes an antibody that binds selectively to the extracellular domain of Her-2/neu.

5. The method of any preceding claim, wherein the immunoassay uses one or two antibodies that bind selectively to the cytoplasmic domain of Her-2/neu.

6. The method of claim 5, wherein the immunoassay uses two antibodies that bind selectively to the cytoplasmic domain of Her-2/neu.

7. The method of any preceding claim, wherein the immunoassay uses a polyclonal antibody against Her-2/neu.

8. The method of any preceding claim, wherein the immunoassay uses a monoclonal antibody against Her-2/neu, preferably wherein the monoclonal antibody is a humanized mouse monoclonal antibody, more preferably wherein the monoclonal antibody is trastuzumab.

9. The method of any preceding claim, wherein the cancer cells are epithelial cells.

10. A method of identifying a cancer patient likely to benefit from treatment with an anticancer agent that targets Her-2/neu, comprising the method of any one of claims 1 to 9, wherein the cancer cell-containing blood sample is a sample from the patient.

11. The method of claim 10, wherein a tumor biopsy tissue from the patient has been previously determined to be negative for Her-2/neu expression by a tissue assay for Her-2/neu.

12. The method of claim 11, wherein the tissue assay is selected from the list consisting of immunohistochemistry and FISH analysis.

## Patentansprüche

1. Verfahren zum Nachweis der Expression von Her-2/neu-Protein auf zirkulierenden Krebszellen in einer Blutprobe, umfassend das Isolieren der Krebszellen aus der Blutprobe, das Lysieren der isolierten Krebszellen, um ein Zellysat zu ergeben, und anschließend Durchführen eines Immuntests mit dem Zellysat, der in der Lage ist, Krebszell-assoziiertes Her-2/neu nachzuweisen, wobei ein positives Immuntestergebnis die Gegenwart von Her-2/neu auf den Krebszellen anzeigt;
wobei die Krebszellen durch Zusammenbringen des Bluts mit immunmagnetischen Beads isoliert werden, die in der Lage sind, selektiv an die Krebszellen zu binden; wobei der Immuntest die Nachweistechnik der Elektrochemilumineszenz verwendet; wobei der Immuntest zwei Antikörper verwendet, die selektiv an Her-2/neu binden; und wobei der Immuntest eine folgendermaßen definierte Empfindlichkeit besitzt:
a) in der Lage zu sein, Krebzell-assoziiertes Her-2/neu auf einem Niveau zwischen einem Zehntel und zwanzig Picogramm Her-2/neu pro Milliliter der Blutprobe nachzuweisen; oder
b) in der Lage zu sein, Her-2/neu aus SK-BR-3-Brustkrebszellen nachzuweisen, wenn sie in Blut bei einer Konzentration von weniger als oder gleich 100 SK-BR-3 Zellen pro Milliliter Blut ausgeschüttet werden.

2. Verfahren nach Anspruch 1, wobei das Empfindlichkeitsniveau in a) zwischen einem und zwanzig Picogramm Her-2/neu pro Milliliter Blutprobe liegt, vorzugsweise wobei das Empfindlichkeitsniveau in a) zwischen einem und zehn Picogramm Her-2/neu pro Milliliter der Blutprobe liegt, und stärker bevorzugt, wobei das Empfindlichkeitsniveau in a) zwischen einem und fünf Picogramm Her-2/neu pro Milliliter der Blutprobe liegt.

3. Verfahren nach Anspruch 1 oder Anspruch 2, wobei das Empfindlichkeitsniveau in b) weniger als oder gleich 10 SK-BR-3 Zellen pro Milliliter Blut beträgt, vorzugsweise wobei das Empfindlichkeitsniveau in b) weniger als oder gleich 3 SK-BR-3 Zellen pro Milliliter Blut beträgt, stärker bevorzugt wobei das Empfindlichkeitsniveau in b) weniger als oder gleich 1 SK-BR-3 Zelle pro Milliliter Blut beträgt.

4. Verfahren nach einem vorhergehenden Anspruch, wobei der Immuntest einen Antikörper verwendet, der selektiv an die extrazelluläre Domäne von Her-2/neu bindet.

5. Verfahren nach einem vorhergehenden Anspruch, wobei der Immuntest einen oder zwei Antikörper verwendet, die selektiv an die cytoplasmatische Domäne von Her-2/neu binden.

6. Verfahren Anspruch 5, wobei der Immuntest zwei Antikörper verwendet, die selektiv an die cytoplasmatische Domäne von Her-2/neu binden.

7. Verfahren nach einem vorhergehenden Anspruch, wobei der Immuntest einen polyklonalen Antikörper gegen Her-2/neu verwendet.

8. Verfahren nach einem vorhergehenden Anspruch, wobei der Immuntest einen monoklonalen Antikörper gegen Her-2/neu verwendet, wobei der monoklonale Antikörper vorzugsweise ein humanisierter Maus-monoklonaler Antikörper ist, wobei der monoklonale Antikörper stärker bevorzugt trastuzumab ist.

9. Verfahren nach einem vorhergehenden Anspruch, wobei die Krebszellen Epithelzellen sind.

10. Verfahren zur Identifizierung eines Krebspatienten, der wahrscheinlich von der Behandlung mit einem Antikrebsmittel gegen Her-2/neu profitiert, umfassend das Verfahren nach einem der Ansprüche 1 bis 9, wobei die krebszellhaltige Blutprobe eine Probe aus dem Patienten ist.

11. Verfahren nach Anspruch 10, wobei ein Tumor-Biopsiegewebe aus dem Patienten zuvor als negativ für die Her-2/neu-Expression durch einen Gewebetest auf Her-2/neu bestimmt wurde.

12. Verfahren nach Anspruch 11, wobei der Gewebetest aus der Liste ausgewählt ist, bestehend aus Immunhistochemie und FISH-Analyse.

## Revendications

1. Procédé de détection de l'expression de la protéine Her-2/Neu sur des cellules cancéreuses circulantes dans un échantillon de sang comprenant l'isolement des cellules cancéreuses à partir de l'échantillon de sang, la lyse des cellules cancéreuses isolées pour produire un lysat cellulaire, ceci suivi de la réalisation sur le lysat cellulaire d'un test immunologique capable de détecter Her-2/neu associée aux cellules cancéreuses, un résultats positif du test immunologique indiquant la présence de Her-2/neu sur les cellules cancéreuses ;
dans lequel les cellules cancéreuses sont isolées par la mise en contact du sang avec des billes immunomagnétiques capables de se lier sélectivement aux cellules cancéreuses ;
dans lequel le test immunologique utilise la technique de détection d'électrochimioluminescence ;
dans lequel le test immunologique utilise deux anticorps qui se lient sélectivement à Her-2/neu ;
et dans lequel le test immunologique a une sensibilité définie par :
a) une capacité de détection de Her-2/neu associée aux cellules cancéreuses à un taux entre un dixième et vingt picogrammes de Her-2/neu par millilitre de l'échantillon de sang ; ou
b) une capacité de détection de Her-2/neu à partir de cellules de cancer du sein SK-BR-3 lorsqu'elles sont surchargées dans le sang à une concentration inférieure ou égale à 100 cellules SK-BR-3 par millilitre de sang.

2. Procédé selon la revendication 1, dans lequel le niveau de sensibilité dans a) se situe entre un et vingt picogrammes de Her-2/neu par millilitre de l'échantillon de sang, de préférence dans lequel le niveau de sensibilité dans a) se situe entre un et dix picogrammes de Her-2/neu par millilitre de l'échantillon de sang, et de manière davantage préférée dans lequel le niveau de sensibilité dans a) se situe entre un et cinq picogrammes de Her-2/neu par millilitre de l'échantillon de sang.

3. Procédé selon la revendication 1 ou 2, dans lequel le niveau de sensibilité dans b) est inférieur ou égal à 10 cellules SK-BR-3 par millilitre de sang, de préférence dans lequel le niveau de sensibilité dans b) est inférieur ou égal à 3 cellules SK-BR-3 par millilitre de sang, de manière davantage préférée dans lequel le niveau de sensibilité dans b) est inférieur ou égal à 1 cellule SK-BR-3 par millilitre de sang.

4. Procédé selon l'une quelconque des revendications précédentes, dans lequel le test immunologique utilise un anticorps qui se lie sélectivement au domaine extracellulaire de Her-2/neu.

5. Procédé selon l'une quelconque des revendications précédentes, dans lequel le test immunologique utilise un ou deux anticorps qui se lient sélectivement au domaine cytoplasmique de Her-2/neu.

6. Procédé selon la revendication 5, dans lequel le test immunologique utilise deux anticorps qui se lient sélectivement au domaine cytoplasmique de Her-2/neu.

7. Procédé selon l'une quelconque des revendications précédentes, dans lequel le test immunologique utilise un anticorps polyclonal dirigé contre Her-2/neu.

8. Procédé selon l'une quelconque des revendications précédentes, dans lequel le test immunologique utilise un anticorps monoclonal dirigé contre Her-2/neu, de préférence dans lequel l'anticorps monoclonal est un anticorps monoclonal de souris humanisé, de manière davantage préférée dans lequel l'anticorps monoclonal est le trastuzumab.

9. Procédé selon l'une quelconque des revendications précédentes, dans lequel les cellules cancéreuses sont des cellules épithéliales.

10. Procédé d'identification d'un patient cancéreux susceptible de bénéficier d'un traitement avec un agent anticancéreux qui cible Her-2/neu, comprenant le procédé selon l'une quelconque des revendications 1 à 9, dans lequel l'échantillon de sang contenant des cellules cancéreuses est un échantillon provenant du patient.

11. Procédé selon la revendication 10, dans lequel un tissu de biopsie tumorale provenant du patient a été précédemment déterminé comme étant négatif pour l'expression de Her-2/neu par un test tissulaire pour Her-2/neu.

12. Procédé selon la revendication 11, dans lequel le test tissulaire est choisi dans la liste comprenant une analyse d'immunohistochimie et FISH.
